# EUROPÄISCHE PATENTSCHRIFT

(11) **EP 0 683 166 B1**
(45) Veröffentlichungstag und Bekanntmachung des Hinweises auf die Patenterteilung: **28.10.1998**
(21) Anmeldenummer: 95105227.3
(22) Anmeldetag: 07.04.1995
(51) Int. Cl.: C07D 401/14, A61K 31/445

(54) **3-Indolylpiperidine**
3-Indolylpiperidines
3-Indolylpipéridines

(30) Priorität: 22.04.1994 DE 4414113
(43) Veröffentlichungstag der Anmeldung: 22.11.1995
(73) Patentinhaber: MERCK PATENT GmbH, 64293 Darmstadt (DE)
(72) Erfinder: Böttcher, Henning, Dr., D-64287 Darmstadt (DE); März, Joachim, Dr., D-55128 Mainz (DE); Seyfreid, Christoph, Dr., D-64342 Jugenheim (DE); Greiner, Harmut, Dr., D-64331 Weiterstadt (DE); Bartoszyk, Gerd, D-64331 Weiterstadt (DE)

(56) Entgegenhaltungen:
- EP-A- 0 058 975
- EP-A- 0 135 781
- EP-A- 0 177 087
- EP-A- 0 200 322
- DE-A- 2 719 294
- FR-A- 2 675 801
- GB-A- 2 044 254
- GB-A- 2 184 444
- US-A- 5 256 673

## Beschreibung

Die Erfindung betrifft 3-Indolylpiperidine der Formel I 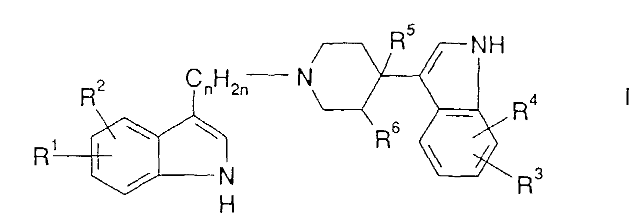 worin
- R¹, R², R³ und R⁴: jeweils unabhängig voneinander H, A, OH, OA, F, Cl, Br, I, CN, CF₃, COOH, CONH₂, CONHA, CONA₂ oder COOA, oder
- R¹ und R² sowie R³ und R⁴: jeweils zusammen auch Methylendioxy,
- R⁵: H oder OH,
- R⁶: H oder
- R⁵ und R⁶: auch gemeinsam eine Bindung,
- A: Alkyl mit 1 bis 6 C-Atomen und
- n: 2, 3, 4, 5 oder 6
bedeuten,
sowie deren physiologisch unbedenkliche Salze.

Der Erfindung lag die Aufgabe zugrunde, neue Verbindungen aufzufinden, die zur Herstellung von Arzneimitteln verwendet werden können. Indol-3-ylmethyl-4-piperidinoderivate sind z.B aus der FR 2 675 801 bekannt. N-(1-(indol-3-ylmethyl)-piperid-4-yl)-1H-dihydro-isoindole sind z.B. in GB 2 044 254 beschrieben.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem serotonin-agonistische und -antagonistische Wirkungen. Sie hemmen die Bindung von tritiierten Serotoninliganden an hippocampale Rezeptoren (Cossery et al., European J. Pharmacol. 140 (1987), 143-155). Außerdem treten Veränderungen der DOPA-Akkumulation im Striatum und der 5-HTP-Akkumulation in N.raphe auf (Seyfried et al., European J. Pharmacol. 160 (1989), 31-41). Weiterhin treten analgetische und blutdrucksenkende Wirkungen auf; so wird bei kathetertragenden wachen, spontan hypertonen Ratten (Stamm SHR/Okamoto/NIH-MO-CHB-Kisslegg; Methode vgl. Weeks und Jones, Proc. Soc. Exptl. Biol. Med. 104 (1960), 646-648) der direkt gemessene Blutdruck nach peroraler Gabe der Verbindungen gesenkt. Ebenso eignen sie sich als Prophylaxe und zur Bekämpfung der Folgen cerebraler Infarktgeschehen (Apoplexia cerebri), wie Schlaganfall und cerebraler Ischämien sowie zur Behandlung extrapyramidal-motorischer Nebenwirkungen von Neuroleptika und des Morbus Parkinson.

Verbindungen der Formel I und ihre physiologisch unbedenklichen Säureadditionssalze können daher als Arzneimittelwirkstoffe für Anxiolytika, Antidepressiva und/oder Antihypertonika und auch als Zwischenprodukte zur Herstellung anderer Arzneimittelwirkstoffe verwendet werden.

Gegenstand der Erfindung sind die Indolderivate der Formel I sowie ihre physiologisch unbedenklichen Säureadditionssalze.

Der Rest A bedeutet Alkyl mit 1, 2, 3, 4, 5 oder 6, insbesondere 1 oder 2 C-Atomen, vorzugsweise Methyl, ferner auch Ethyl, n-Propyl, Isopropyl, n-Butyl, Isobutyl, sek.-Butyl oder tert.-Butyl. OA ist vorzugsweise Methoxy, ferner auch Ethoxy, n-Propoxy, Isopropoxy, n-Butoxy, Isobutoxy, sek.-Butoxy oder tert.-Butoxy. NHA ist vorzugsweise Methylamino, ferner Ethylamino, n-Propylamino, Isopropylamino, n-Butylamino, Isobutylamino, sek.-Butylamino oder tert.-Butylamino. NA₂ bedeutet vorzugsweise Dimethylamino, ferner N-Ethyl-N-methylamino, Diethylamino, Di-n-propylamino, Diisopropylamino oder Di-n-butylamino.

Daraus resultierend bedeutet CO-NHA besonders bevorzugt N-Methylcarbamoyl oder N-Ethylcarbamoyl und CO-NA₂ vorzugsweise N,N-Dimethylcarbamoyl oder N,N-Diethylcarbamoyl.

Die Indolreste sind unsubstituiert oder ein- oder zweifach substituiert. Die einfache Substitution ist bevorzugt, wobei sich die Substituenten vorzugsweise in 5-Stellung befinden, ferner aber auch in der 2-, 4-, 6- oder 7-Stellung sein können. Sofern die beiden Indol-3-yl-Reste substituiert sind, können die jeweiligen Substituenten gleich oder verschieden sein. Ebenso kann die Anzahl der Substituenten beider Indolreste verschieden voneinander sein.

Bevorzugte Substituenten R¹, R², R³ und R⁴ an den Indolylresten sind beispielsweise CO₂H, CO₂CH₃, OCH₃, OH, O-CH₂-O, F, CN oder CONH₂.

Sofern das Indolsystem in 2-Stellung substituiert ist, so ist dort eine Substitution durch A besonders bevorzugt.

Der Parameter n kann 2, 3, 4, 5 oder 6 bedeuten, vorzugsweise ist er 3 oder 4.

R⁵ und R⁶ bedeuten bevorzugt jeweils Wasserstoff, können ferner aber auch zusammen eine Bindung bedeuten.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können auch durch die nachstehenden Formeln la bis Ih ausgedrückt werden, die der Formel I entsprechen und worin die nicht näher bezeichneten Reste die bei der Formel I angegebene Bedeutung haben, worin jedoch
- in Ia: R² und R⁴ H bedeuten und R¹ und R³ gleich sind und sich jeweils in 5-Position der Indolreste befinden;
- in Ib: R² und R⁴ H bedeuten und R¹ und R³ jeweils COOH, COOA, CONH₂, CONHA, CONA₂ oder CN bedeuten und sich jeweils in 5-Position der Indolreste befinden;
- in Ic: R² und R⁴ H bedeuten und R¹ und R³ jeweils OH, OA, F, Cl, Br, I oder CF₃ bedeuten und sich jeweils in 5-Position der Indolreste befinden;
- in Id: R¹ und R² sowie R³ und R⁴ jeweils zusammen Methylendioxy bedeuten;
- in Ie: R², R⁴, R⁵ und R⁶ jeweils H bedeuten;
- in If: R² und R⁴ H und R⁵ und R⁶ zusammen eine Bindung bedeuten;
- in Ig: R², R⁴, R⁵ und R⁶ jeweils H bedeuten und R¹ sowie R³ gleich sind und F, CN, OA oder CONH₂ bedeuten:
- in Ih: R², R⁴, R⁵ und R⁶ jeweils H bedeuten und R¹ sowie R³ verschieden voneinander sind und jeweils H, COOH, COOA, OCH₃, OH, CN, CONH₂, CONA₂ oder CONHA bedeuten;
- in Ii: R¹ und/oder R³ A, insbesonder Methyl, bedeuten und sich in 2-Position der Indolreste befinden.

Insbesondere sind jedoch solche Verbindungen der Teilformeln Ik sowie Iak bis Ihk bevorzugt, die den Teilformeln Ia bis Ik und der Formel I entsprechen, worin jedoch zusätzlich n 2, 3 oder 4 bedeutet.

Gegenstand der Erfindung ist ferner ein Verfahren zur Herstellung von Piperidinderivaten der Formel I sowie von deren Salzen, dadurch gekennzeichnet, daß man eine Verbindung der Formel II 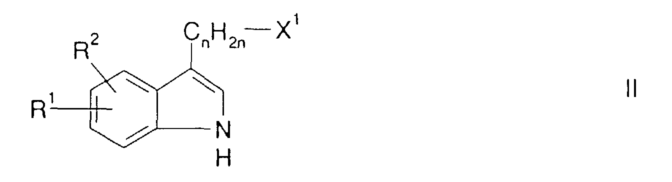 worin
- X¹: X oder NH₂ und
- X: Cl, Br, I, OH oder eine reaktionsfähig funktionell abgewandelte OH-Gruppe bedeuten und
R¹, R² und n die angegebenen Bedeutungen haben, mit einer Verbindung der Formel III 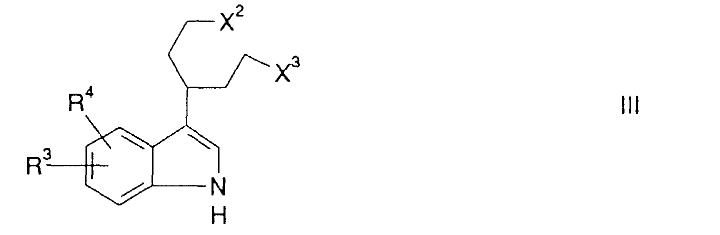 worin
- X² und X³: gleich oder verschieden sein können und, falls X¹ = NH₂ ist, jeweils X, andernfalls zusammen NH bedeuten und
- R³ und R⁴: die angegebenen Bedeutungen haben,
umsetzt,
oder daß man eine Verbindung der Formel IV 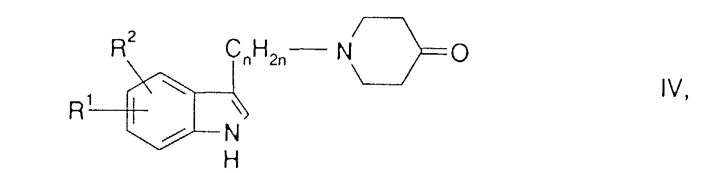 worin R¹, R² und n die angegebenen Bedeutungen haben, mit einem Indol der Formel V 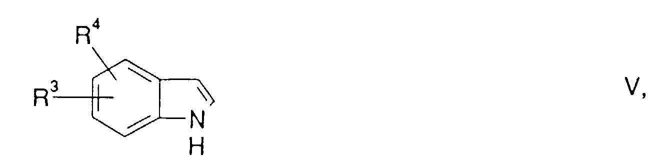 worin R³ und R⁴ die angegebenen Bedeutungen besitzen,
umsetzt,
oder daß man eine Verbindung der Formel I, in der R⁵ OH und R⁶ H bedeutet, durch Dehydratisierung in eine andere Verbindung der Formel I umwandelt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C-und/oder C-N-Bindung(en) enthält, mit einem reduzierenden Mittel behandelt
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man einen Rest R¹, R², R³ und/oder R⁴ durch Veresterung, Verseifung, Veretherung, Etherspaltung, vollständige oder partielle Hydrolyse oder durch Alkylierung in (einen) andere(n) Rest(e) R¹, R², R³ und/oder R⁴ umwandelt
und/oder daß man eine erhaltende Base oder Säure der Formel I durch Behandeln mit einer Säure oder Base in eines ihrer Salze umwandelt.

Die Herstellung der Verbindungen der Formel I erfolgt im übrigen nach an sich bekannten Methoden, wie sie in der Literatur (z.B. in Standardwerken wie Houben-Weyl, Methoden der Organischen Chemie, Georg-Thieme-Verlag, Stuttgart; Organic Reactions, John Wiley & Sons, Inc. New York; J. March, Adv. Org. Chem., 3rd Ed. J. Wiley & Sons (1985)) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, daß man sie aus dem Reaktionsgemisch nicht isoliert, sondern sofort weiter zu den Verbindungen der Formel I umsetzt.

In den Indolderivaten der Formel II ist X¹ vorzugsweise X; dementsprechend sind in den Verbindungen der Formel II X² und X³ vorzugsweise zusammen NH. Der Rest X ist vorzugsweise Cl oder Br; er kann jedoch auch 1, OH oder eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsulfonyloxy, p-Toluolsulfonyloxy, 1- oder 2-Naphthalin-sulfonyloxy).

Dementsprechend sind die Indolderivate der Formel I insbesondere durch Umsetzung von 3-(Chloralkyl)- oder 3-(Bromalkyl)-indolen mit 3-Piperid-4-yl-indolen, worin demnach X² und X³ zusammen eine NH-Gruppe bedeuten (nachstehend als IIIa bezeichnet) erhältlich.

Die Verbindungen der Formeln II und insbesondere III sind zum Teil bekannt; die nicht bekannten Verbindungen der Formeln II und III können leicht analog zu den bekannten Verbindungen hergestellt werden.

Primäre Alkohole der Formel Ind-CₙH₂ₙ-OH, wobei Ind stets für den durch R¹ und R² bzw. R³ und R⁴ substituierten Indol-3-yl-Rest steht, sind z.B. durch Reduktion der entsprechenden Carbonsäuren oder ihrer Ester erhältlich. Behandeln mit Thionylchlorid, Bromwasserstoff, Phosphortribromid oder ähnlichen Halogenverbindungen liefert die entsprechenden Halogenide der Formel Ind-CₙH₂ₙ-Hal (Hal: Br, Cl). Die entsprechenden Sulfonyloxyverbindungen sind erhältlich aus den Alkoholen Ind-CₙH₂ₙ-OH durch Umsetzung mit den entsprechenden Sulfonsäurechloriden.

Die lodverbindungen der Formel I Ind-CₙH₂ₙ-I sind z.B. durch Einwirkung von Kaliumiodid auf die zugehörigen p-Toluolsulfonsäureester erhältlich. Die Amine der Formel Ind-CₙH₂ₙ-NH₂ sind z.B. aus den Halogeniden mit Phthalimidkalium oder durch Reduktion der entsprechenden Nitrile herstellbar.

Die Piperidinderivate IIIa sind größtenteils bekannt und z.B. erhältlich durch Umsetzung von in 1-Position durch übliche, an sich bekannte Aminoschutzgruppen geschütztes 4-Piperidinon, mit Indolen, die gegebenenfalls durch die Reste R³ und/oder R⁴ substituiert sind. Vorzugsweise führt man diese Reaktionen unter Einwirkung eines Katalysators, beispielsweise oiner Säure, durch. Das entstandene Produkt kann dann, nach Entfemung der Schutzgruppe direkt mit einer Verbindung der Formel II umgesetzt werden, oder aber vorher zu einem 1 ,2,5,6-Tetrahydropyridinderivat dehydratisiert bzw. anschließend noch hydriert werden.

Die Umsetzung der Verbindungen II und III verläuft nach Methoden, wie sie für die elektrophile Substitution an Indolen aus der Literatur bekannt sind. Man kann ohne Gegenwart eines Lösungsmittels die Komponenten miteinander zur Reaktion bringen, gegebenenfalls im geschlossenen Rohr oder im Autoklaven. Es ist aber bevorzugt, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, n-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser.

Die Reaktionszeit liegt je nach den angewendeten Bedingungen zwischen einigen Minuten und 14 Tagen, die Reaktionstemperatur zwischen etwa 0 und 150 °C, normalerweise zwischen 20 und 130 °C.

In einigen Fällen kann der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin die Reaktion begünstigen. In anderen Fällen ist der Zusatz katalytischer Mengen einer Säure, vorzugsweise einer Mineralsäure wie z.B. HCI, günstig.

Ferner ist es möglich eine Verbindung der Formel I zu erhalten, indem man eine Verbindung der Formel IV mit einem Indolderivat der Formel V umsetzt.

Die Verbindungen der Formeln IV und insbesondere V sind zum Teil bekannt; die nicht bekannten Verbindungen können leicht in Analogie zu den bekannten hergestellt werden. So lassen sich Verbindungen der Formel IV leicht durch Umsetzung von Ind-CₙH₂ₙ-NH₂ mit 1 ,5-Dihalogenpentanon-3, wobei Halogen bevorzugt für Chlor oder Brom steht, herstellen. Ebenso ist es möglich, Verbindungen des Typs IV durch Umsetzung von Ind-CₙH₂ₙ-Cl, Ind-CₙH₂ₙ-Br oder Ind-CₙH₂ₙ-I mit 4-Piperidon zu erhalten.

Die Indole der Formel V lassen sich durch die diversen, an sich bekannten Möglichkeiten der Indolsynthese, beispielsweise der Fischer-Indolsynthese, herstellen.

Die Umsetzung der Verbindungen IV und V verläuft nach Methoden, wie sie für die Reaktionen von Enaminen mit elektrophilen Reaktionspartnern aus der Literatur bekannt sind. Die Komponenten können direkt ohne Gegenwart eines Lösungsmittels, miteinander umgesetzt werden, gegebenenfalls im geschlossenen Rohr oder im Autoklaven, unter Normaldruck oder unter erhöhtem Druck, wobei ein Inertgas wie z.B. N₂ zur Druckerhöhung zugeführt wird. Es ist aber auch möglich, die Verbindungen in Gegenwart eines inerten Lösungsmittels umzusetzen. Als Lösungsmittel eignen sich die zuvor bei der Umsetzung von II mit III genannten.

Die optimale Reaktionszeit liegt, je nach den gewählten Reaktionsbedingungen, zwischen einigen Minuten und 14 Tagen, die Reaktionstemperatur zwischen etwa 0° und 150°, üblicherweise zwischen 20° und 130°.

Es ist ferner möglich, eine Verbindung der Formel I zu erhalten, indem man ein Vorprodukt, das anstelle von Wasserstoffatomen eine oder mehrere reduzierbare Gruppe(n) und/oder eine oder mehrere zusätzliche C-C- und/oder C-N-Bindung(en) enthält, mit einem reduzierenden Mittel behandelt, vorzugsweise bei Temperaturen zwischen -80 und +250° in Gegenwart mindestens eines inerten Lösungsmittels.

Reduzierbare (durch Wasserstoff ersetzbare) Gruppen sind insbesondere Sauerstoff in einer Carbonylgruppe, Hydroxyl, Arylsulfonyloxy (z.B. p-Toluolsulfonyloxy), N-Benzolsulfonyl, N-Benzyl oder O-Benzyl.

Es ist grundsätzlich möglich, Verbindungen, die nur eine, oder solche, die nebeneinander zwei oder mehr der oben angeführten Gruppen bzw. zusätzlichen Bindungen enthalten, reduktiv in eine Verbindung der Formel I überzuführen; dabei können gleichzeitig Substituenten in der Indolreste, die in der Ausgangsverbindung enthalten sind, reduziert werden. Vorzugsweise bedient man sich hierzu des nascierenden Wasserstoffs oder komplexer Metallhydride, ferner der Reduktion nach Wolff-Kishner sowie der Reduktionen mit Wasserstoffgas unter Übergangsmetallkatalyse.

Bevorzugte Ausgangsstoffe für die Reduktion entsprechen der Formel VI 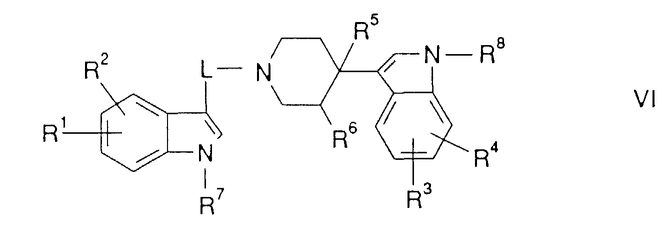 worin
- R⁷ und R⁸: H bedeuten oder beispielsweise für Arylsulfonylgruppen und/oder Benzylgruppen stehen,
- L: CₙH₂ₙ oder eine diesem Rest entsprechende Kette, worin jedoch eine oder mehrere -CH₂-Gruppe(n) durch -CO-ersetzt sind,
bedeuten
und R¹, R², R³, R⁴, R⁵ und R⁶ die angegebenen Bedeutungen haben,
worin jedoch R⁷ und R⁸ nicht gleichzeitig H und L CₙH₂ₙ sein können.

In den Verbindungen der Formel VI ist L bevorzugt -CO-(CH₂)ₙ₋₂-CO- [im einzelnen -COCO-, -COCH₂CO-, -CO-(CH₂)₂-CO-, -CO-(CH₂)₃-CO-], -(CH₂)ₙ₋₁-CO- [im einzelnen -CH₂-CO-, -CH₂CH₂-CO-, -(CH₂)₃-CO- oder -(CH₂)₄-CO-], ferner z.B. -CO-CH₂CH₂-, -CO-(CH₂)₃-, -CH₂-CO-CH₂CH₂-, -CH₂CH₂-CO-CH₂-, -CO-(CH₂)₄-, -CH₂-CO-(CH₂)₃-, -CH₂CH₂-CO-CH₂CH₂- oder -(CH₂)₃-CO-CH₂-.

Verbindungen der Formel VI sind z.B. herstellbar durch Umsetzung von Verbindungen der Formel III, die gegebenenfalls vorher in 1-Position substituiert wurden, mit einer Verbindung der Formel VII 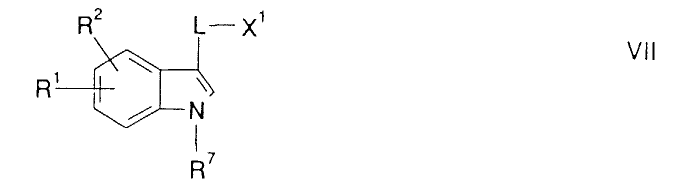 worin R¹, R², R⁷, L und X¹ die oben angegebenen Bedeutungen haben, unter den Bedingungen, die zuvor für die Umsetzung von II mit III angegeben sind.

Wird als Reduktionsmittel nascierender Wasserstoff verwendet, so kann man diesen z.B. durch Behandlung von Metallen mit schwachen Säuren oder mit Basen erzeugen. So kann man z.B. ein Gemisch von Zink mit Alkalilauge oder von Eisen mit Essigsäure verwenden. Geeignet ist auch die Verwendung von Natrium oder einem anderen Alkalimetall in einem Alkohol wie Ethanol, Isopropanol, Butanol, Amyl- oder Isoamylalkohol oder Phenol. Man kann ferner eine Aluminium-Nickel-Legierung in alkalisch-wässeriger Lösung, gegebenenfalls unter Zusatz von Ethanol, verwenden. Auch Natrium- oder Aluminiumamalgam in wässerig-alkoholischer oder wässeriger Lösung sind zur Erzeugung des nascierenden Wasserstoffs geeignet. Die Umsetzung kann auch in heterogener Phase durchgeführt werden, wobei man zweckmäßig eine wässerige und eine Benzol- oder Toluol-Phase verwendet.

Als Reduktionsmittel können ferner besonders vorteilhaft komplexe Metallhydride, wie LiAlH₄, NaBH₄, Disiobutylaluminiumhydrid oder NaAl(OCH₂CH₂OCH₃)₂H₂ sowie Diboran eingesetzt werden, falls erwünscht unter Zusatz von Katalysatoren wie BF₃, AlCl₃ oder LiBr. Als Lösungsmittel eignen sich hierfür insbesondere Ether wie Diethylether, Di-n-butylether, THF, Dioxan, Diglyme oder 1,2-Dimethoxyethan sowie Kohlenwasserstoffe wie Benzol. Für eine Reduktion mit NaBH₄ sind in erster Linie Alkohole wie Methanol oder Ethanol, ferner Wasser sowie wässerige Alkohole als Lösungsmittel geeignet. Nach diesen Methoden reduziert man vorzugsweise bei Temperaturen zwischen -80 und +150°, insbesondere zwischen etwa 0 und etwa 100°.

Besonders vorteilhaft lassen sich -CO-Gruppen in Säureamiden (z.B. solchen der Formel VI, worin L eine -(CH₂)ₙ₋₁-CO-Gruppe bedeutet) mit LiAlH₄ in THF bei Temperaturen zwischen etwa 0 und 66° zu CH₂-Gruppen reduzieren. Dabei können in 1-Stellung des Indolring befindliche Arylsulfonyl-Schutzgruppen gleichzeitig reduktiv abgespalten werden. N-Benzylgruppen können reduktiv mit Natrium in flüssigem Ammoniak abgespalten werden.

Es ist ferner möglich, eine oder mehrere Carbonylgruppen nach der Methode von Wolff-Kishner zu CH₂-Gruppen zu reduzieren, z.B. durch Behandlung mit wasserfreiem Hydrazin in absolutem Ethanol unter Druck bei Temperaturen zwischen etwa 150 und 250°. Als Katalysator wird vorteilhaft Natriumalkoholat verwendet. Die Reduktion kann auch nach der Methode von Huang-Minlon variiert werden, indem man mit Hydrazinhydrat in einem hochsiedenden, mit Wasser mischbaren Lösungsmittel, wie Diethylenglykol oder Triethylenglykol, in Gegenwart von Alkali, wie Natriumhydroxiod, umsetzt. Das Reaktionsgemisch wird in der Regel etwa 3-4 Stunden gekocht. Anschließend wird das Wasser abdestilliert und das gebildete Hydrazon bei Temperaturen bis zu etwa 200° zersetzt. Die Wolff-Kishner-Reduktion kann auch bei Raumtemperatur in Dimethylsulfoxid mit Hydrazin ausgeführt werden.

Darüber hinaus ist es möglich, bestimmte Reduktionen durch Verwendung von H₂-Gas unter katalytischer Wirkung von Übergangsmetallen, wie z.B. Raney-Ni oder Pd durchzuführen. Man kann auf diese Weise z.B. Cl, Br, I, SH oder in bestimmten Fällen auch OH-Gruppen durch Wasserstoff ersetzen. Ebenso können Nitrogruppen durch katalytische Hydrierung mit Pd/H₂ in Methanol in NH₂-Gruppen umgewandelt werden.

Verbindungen, die sonst der Formel I entsprechen, aber anstelle eines oder mehrerer H-Atome eine oder mehrere solvolysierbare Gruppe(n) enthalten, können zu den Verbindungen der Formel I solvolysiert, insbesondere hydrolysiert werden.

Die Ausgangsstoffe für die Solvolyse sind beispielsweise erhältlich durch Reaktion von IIIa mit Verbindungen, die der Formel II (X¹ = X) entsprechen, aber anstelle eines oder mehrerer H-Atome eine oder mehrere solvolysierbare Gruppe(n) enthalten. So können insbesondere 1-Acylindolderivate (entsprechend der Formel I, aber in 1-Stellung des Indol-Rests eine Acylgruppe enthaltend, vorzugsweise eine Alkanoyl-, Alkylsulfonyl- oder Arylsulfonylgruppe mit jeweils bis zu 10 C-Atomen, wie Methan-, Benzol- oder p-Toluolsulfonyl) zu den entsprechenden in der 1-Stellung des Indolringes unsubstituierten Indolderivaten hydrolysiert werden, z.B. in saurem, besser in neutralem oder alkalischem Medium bei Temperaturen zwischen 0 und 200°. Als Basen verwendet man zweckmäßig Natrium-, Kalium- oder Calciumhydroxid, Natrium- oder Kaliumcarbonat oder Ammoniak. Als Lösungsmittel wählt man vorzugsweise Wasser; niedere Alkohole wie Methanol, Ethanol; Ether wie THF, Dioxan; Sulfone wie Tetramethylensulfon; oder deren Gemische, besonders die Wasser enthaltenden Gemische. Eine Hydrolyse kann auch bereits beim Behandeln mit Wasser allein erfolgen, insbesondere in der Siedehitze.

Weiterhin kann man eine Verbindung der Formel I nach an sich bekannten Methoden in eine andere Verbindung der Formel I umwandeln.

Verbindungen der Formel I, worin das Indol-System z.B. durch COOA, CONH₂, CONHA, CONA₂ substituiert ist, können durch Derivatisierung entsprechender Carboxy-indol-3-yl-Verbindungen erhalten werden. Man kann z.B. die Säuren oder ihre reaktionsfähigen Derivate, wie z.B. ihre Säurehalogenide oder Anhydride mit entsprechenden Alkoholen oder Alkoholaten, unter Verwendung der an sich bekannten Methoden oder einer der zahlreichen Varianten, verestern. Femer ist es möglich, Säuren, Säurehalogenide oder Ester mit primären oder sekundären, aliphatischen oder cyclischen Aminen zu amidieren. Bevorzugt ist die Umsetzung der freien Carbonsäure mit dem Amin unter den Bedingungen einer Peptidsynthese. Diese Reaktion gelingt vorzugsweise in Gegenwart eines Dehydratisierungsmittels, z.B. eines Carbodiimids wie Dicyclohexylcarbodiimid oder N-(3-Dimethylaminopropyl)-N-ethyl-carbodiimid, ferner Propanphosphonsäureanhydrid (vgl. Angew. Chemie 92, 129 (1980)), Diphenylphosphorylazid oder 2-Ethoxy-N-ethoxycarbonyl-1 ,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40°, vorzugsweise zwischen 0 und 30°. Anstelle der Säure bzw. des Amids können auch reaktionsfähige Derivate dieser Stoffe in die Reaktion eingesetzt werden, z.B. solche, in denen reaktive Gruppen intermediär durch Schutzgruppen blockiert sind. Die Säuren können auch in Form ihrer aktivierten Ester verwendet werden, die zweckmäßig in situ gebildet werden, z.B. durch Zusatz von 1-Hydroxybenztriazol oder N-Hydroxysuccinimid.

Weiterhin kann man cyan-substituierte Indol-3-yl-Reste zu Carboxy-indol-3-yl- oder Carbamido-indol-3-yl-Resten hydrolysieren.

Verbindungen der Formel I, in denen die Indol-Reste ein- oder zweifach durch O-Alkyl substituiert sind, können einer Etherspaltung unterzogen werden, wobei die entsprechenden Hydroxyderivate entstehen. Z.B. kann man die Ethergruppen spalten durch Behandeln mit Dimethylsulfid-Bortribromid-Komplex, z.B. in Toluol, Ethern wie THF oder Dimethylsulfoxid, oder durch Verschmelzen mit Pyridin- oder Anilinhydrohalogeniden, vorzugsweise Pyridinhydrochlorid, bei etwa 150-250°.

Die Verbindungen der Formel I können gegebenenfalls ein Asymmetriezentrum besitzen. Sie können daher bei ihrer Herstellung als Racemate oder, falls optisch aktive Ausgangsstoffe verwendet werden, auch in optisch aktiver Form erhalten werden. Erhaltene Racemate können, falls erwünscht, nach an sich bekannten Methoden mechanisch oder chemisch in ihre optischen Antipoden getrennt werden. Vorzugsweise werden aus dem Racemat durch Umsetzung mit einem optisch aktiven Trennmittel Diastereomere gebildet. Als Trennmittel eignen sich z.B. optisch aktive Säuren, wie die D- und L-Formen von Weinsäuren, Dibenzoylweinsäure, diacetylweinsäure, Camphersulfonsäuren, Mandelsäure, Äpfelsäure oder Milchsäure. Die verschiedenen Formen der Diastereomeren können in an sich bekannter Weise, z.B. durch fraktionierte Kristallisation, getrennt, und die optisch aktiven Verbindungen der Formel I können in an sich bekannter Weise aus den Diastereomeren in Freiheit gesetzt werden.

Eine erhaltene Base der Formel 1 kann mit einer Säure in das zugehörige Säureadditionssalz übergeführt werden. Für diese Umsetzung eignen sich Säuren, die physiologisch unbedenkliche Salze liefem. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetallhydroxide oder organische Basen in Form von primären, sekundären oder tertiären Aminen.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze. Diese Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlenhydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B. ein oder mehrere Vitamine.

Die Verbindungen der Formel I und ihre physiologisch unbedenklichen Salze können bei der therapeutischen Behandlung des menschlichen oder tierischen Körpers und bei der Bekämpfung von Krankheiten verwendet werden. Sie eignen sich zur Behandlung extrapyramidal-motorischer Nebenwirkungen von Neuroleptika, von Erkrankungen des Zentralnervensystems wie Spannungszuständen, Depressionen und/oder Psychosen und von Nebenwirkungen bei der Behandlung der Hypertonie (z.B. mit α-Methyldopa). Ferner können die Verbindungen in der Endokrinologie und Gynäkologie Verwendung finden, z.B. zur Therapie von Akromegalie, Hypogonadismus, sekundärer Amenorrhoe, prämenstruellem Syndrom, unerwünschter puerperaler Laktation, weiterhin zur Prophylaxe und Therapie cerebraler Störungen (z.B. Migräne), insbesondere in der Geriatrie ähnlich wie gewisse Ergot-Alkaloide und zur Bekämpfung der Folgen cerebraler Infarktgeschehen (Apoplexia cerebri), wie Schlaganfall und cerebraler Ischämien.

Dabei werden die erfindungsgemäßen Substanzen in der Regel in Analogie zu bekannten, im Handel befindlichen Präparaten, z.B. Bromocriptin, Dihydroergocornin verabreicht, vorzugsweise in Dosierungen zwischen etwa 0,2 und 500 mg, insbesondere zwischen 0,2 und 50 mg pro Dosierungseinheit. Die tägliche Dosierung liegt vorzugsweise zwischen etwa 0,001 uns 10 mg/kg Körpergewicht. Die niedrigen Dosierungen (etwa 0,2 bis 1 mg pro Dosierungseinheit; etwa 0,001 bis 0,005 mg/kg Körpergewicht) kommen dabei insbesondere für die Verwendung als Migränemittel in Betracht; für die übrigen Indikationen werden Dosierungen zwischen 10 und 50 mg Dosierungseinheit bevorzugt. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welche die Therapie gilt. Die orale Applikation ist bevorzugt.

In den nachstehenden Beispielen bedeutet "übliche Aufarbeitung": Man gibt, falls erforderlich, Wasser hinzu, extrahiert mit Dichlormethan, trennt ab, trocknet die organische Phase über Natriumsulfat, filtriert, dampft ein und reinigt durch Chromatographie an Kieselgel und/oder durch Kristallisation. Temperaturen sind in °C angegeben.

### Beispiel 1

Man löst 1,2 g 3-(4-Chlorbutyl)-5-methoxy-indol [erhältlich durch Umsetzung von 5-Methoxyindol mit 4-Chlorbutyrylchlorid zu 3-(4-Chlorbutyryl)-5-methoxyindol und anschließende Reduktion mit Diboran] sowie 1,0 g 4-(lndol-3-yl)-piperidin [erhältlich durch Umsetzung von N-BOC-4-Piperidon mit Indol, anschließende Dehydratisierung und Hydrierung der entstehenden Doppelbindung sowie Abspaltung der Schutzgruppe] in 200 ml Acetonitril und rührt 8 Stunden bei Raumtemperatur. Nach üblicher Aufarbeitung erhält man 3-[1-(4-(5-Methoxy-indol-3-yl)-butyl)-4-piperidyl]-indol, Hydrochlorid, F. 138-141° (Zers.).

Analog erhält man durch Umsetzung
von 3-(4-Chlorbutyl)-indol-5-carbonsäuremethylester mit 4-(5-Methoxyindol-3-yl)-piperidin:
   3-[1-(4-(5-Methoxycarbonylindol-3-yl)-butyl)-4-piperidyl]-5-methoxyindol, Hydrochlorid, F. 222-224°;
von 3-(4-Chlorbutyl)-indol mit 4-(5-Methoxyindol-3-yl)-piperidin:
   3-[1-(4-(lndol-3-yl)-butyl)-4-piperidyl]-5-methoxyindol, Hydrochlorid, F. 213-216°;
von 3-(4-Chlorbutyl)-5-methoxyindol mit 4-(5,6-Methylendioxyindol-3-yl)piperidin:
   3-[1-(4-(5-Methoxyindol-3-yl)-butyl)-4-piperidyl]-5,6-methylendioxyindol, Hydrochlorid, F. 144-146°;
von 3-(4-Chlorbutyl)-5-methoxy indol mit 4-(5-Methoxyindol-3-yl)-piperidin:
   3-[1-(4-(5-Methoxy-indol-3-yl)-butyl)-4-piperidyl]-5-methoxyindol;
von 3-(4-Chlorbutyl)-indol mit 4-(3-lndolyl)-piperidin:
   3-[1-(4-(Indol-3-yl)-butyl)-4-piperidyl]-indol ;
von 3-(4-Chlorbutyl)-5-methoxyindol mit 4-(5-Hydroxyindol-3-yl)-piperidin:
   3-[1-(4-(5-Methoxy-indol-3-yl)-butyl)-4-piperidyl]-5-hydroxyindol, F. 203-204°;
von 3-(4-Chlorbutyl)-5-cyanindol mit 4-(5-Carbamoylindol-3-yl)-piperidin:
   3-[1-(4-(5-Cyan-indol-3-yl)-butyl)-4-piperidyl]-indol-5-carboxamid, Hemihydrat, F. 227-228;
von 3-(4-Chlorbutyl)-5-cyanindol mit 4-(5-Cyanindol-3-yl)-piperidin:
   3-[1-(4-(5-Cyan-indol-3-yl)-butyl)-4-piperidyl]-5-cyanindol, Dihydrat, F. 95-101°;
von 3-(4-Chlorbutyl)-5-methoxycarbonylindol mit 4-(5-Carbamoyl-indol-3-yl)-piperidin:
   3-[1-(4-(5-Methoxycarbonyl-indol-3-yl)-butyl)-4-piperidyl]-indol-5-carboxamid, Hydrat, F. 228-231°;
von 3-(4-Chlorbutyl)-indol-5-carboxamid mit 4-(5-Carbamoylindol-3-yl)piperidin:
   3-[1-(4-(5-Carbamoyl-indol-3-yl)-butyl)-4-piperidyl]-indol-5-carboxamid, Trihydrochlorid, F. 202-203°;
von 3-(4-Chlorbutyl)-5-fluorindol mit 4-(5-Fluorindol-3-yl)-piperidin:
   3-[1-(4-(5-Fluor-indol-3-yl)-butyl)-4-piperidyl]-5-fluor-indol;
von 3-(4-Chlorbutyl)-indol-5-carbonsäuremethylester mit 4-(5-Methoxycarbonylindol-3-yl)-piperidin:
   3-[1-(4-(5-Methoxycarbonyl-indol-3-yl)-butyl)-4-piperidyl]-indol-5-carbonsäuremethylester;
von 3-(4-Chlorbutyl)-indol-5-carboxamid mit 4-(5-Cyanindol-3-yl)-piperidin:
   3-[1-(4-(5-Carbamoyl-indol-3-yl)-butyl)-4-piperidyl]-5-cyanindol,

### Beispiel 2

Man kocht 0,8 g 3-[1-(4-(5-Methoxycarbonyl-indol-3-yl)-butyl)-4-piperidyl]-indol-5-carbonsäuremethylester [erhältlich nach Beispiel 1] 0,5 Std. mit 100 ml 2n ethanolischer KOH, arbeitet wie üblich auf und erhält 3-[1-(4-(5-Carboxy-indol-3-yl)-butyl)-4-piperidyl]-indol-5-carbonsäure.

Analog erhält man durch Verseifung der entsprechenden Ester ausgehend
von 3-[1-(4-(5-Methoxycarbonylindol-3-yl)-butyl)-4-piperidyl]-5-methoxyindol:
   3-[1-(4-(5-Carboxyindol-3-yl)-butyl)-4-piperidyl]-5-methoxy-indol, Hydrochlorid-Hydrat, F. 248° (Zers.);
von 3-[1-(4-(5-Methoxycarbonyl-indol-3-yl)-butyl)-4-piperidyl]-indol-5-carboxamid:
   3-[1-(4-(5-Carboxy-indol-3-yl)-butyl)-4-piperidyl]-indol-5-carboxamid, Hydrochlorid, F. 282-285°.

### Beispiel 3

Analog Beispiel 1 erhält man ausgehend von 3-(3-Chlorpropyl)-5-methoxyindol [erhältlich durch Umsetzung von 5-Methoxyindol mit 3-Chlorpropionylchlorid zu 3-(3-Chlorpropionyl)-5-methoxyindol und anschließende Reduktion mit Diboran] sowie 1,0 g 4-(lndol-3-yl)-piperidin [erhältlich durch Umsetzung von N-BOC-4-Piperidon mit Indol, anschließende Dehydratisierung und Hydrierung der entstehenden Doppelbindung sowie Abspaltung der Schutzgruppe] nach üblicher Aufarbeitung das 3-[1-(3-(5-Methoxy-indol-3-yl)-propyl)-4-piperidyl]-indol.

### Analog erhält man durch Umsetzung

von 3-(3-Chlorpropyl)-indol-5-carbonsäuremethylester mit 4-(5-Methoxyindol-3-yl)-piperidin:
   3-[1-(3-(5-Methoxycarbonylindol-3-yl)-propyl)-4-piperidyl]-5-methoxyindol;
von 3-(3-Chlorpropyl)-indol mit 4-(5-Methoxyindol-3-yl)-piperidin :
   3-[1-(3-(lndol-3-yl)-propyl)-4-piperidyl]-5-methoxyindol, Hydrochlorid;
von 3-(3-Chlorpropyl)-5-methoxyindol mit 4-(5,6-Methylendioxyindol-3-yl)piperidin:
   3-[1-(3-(5-Methoxyindol-3-yl)-propyl)-4-piperidyl]-5,6-methylendioxyindol;
von 3-(3-Chlorpropyl)-5-methoxyindol mit 4-(5-Methoxyindol-3-yl)-piperidin:
   3-[1-(3-(5-Methoxy-indol-3-yl)-propyl)-4-piperidyl]-5-methoxyindol;
von 3-(3-Chlorpropyl)-indol mit 4-(3-lndolyl)-piperidin :
   3-[1-(3-(Indol-3-yl)-propyl)-4-piperidyl]-indol;
von 3-(3-Chlorpropyl)-5-methoxyindol mit 4-(5-Hydroxyindol-3-yl)-piperidin:
   3-[1-(3-(5-Methoxy-indol-3-yl)-propyl)-4-piperidyl]-5-hydroxyindol;
von 3-(3-Chlorpropyl)-5-cyanindol mit 4-(5-Carbamoylindol-3-yl)-piperidin:
   3-[1-(3-(5-Cyan-indol-3-yl)-propyl)-4-piperidyl]-indol-5-carboxamid;
von 3-(3-Chlorpropyl)-5-cyanindol mit 4-(5-Cyanindol-3-yl)-piperidin:
   3-[1-(3-(5-Cyan-indol-3-yl)-propyl)-4-piperidyl]-5-cyanindol;
von 3-(3-Chlorpropyl)-5-methoxycarbonylindol mit 4-(5-Carbamoyl-indol-3-yl)-piperidin:
   3-[1-(3-(5-Methoxycarbonyl-indol-3-yl)-propyl)-4-piperidyl]-indol-5-carboxamid, F. 195-196°;
von 3-(3-Chlorpropyl)-indol-5-carboxamid mit 4-(5-Carbamoylindol-3-yl)-piperidin:
   3-[1-(3-(5-Carbamoyl-indol-3-yl)-propyl)-4-piperidyl]-indol-5-carboxamid, Sesquihydrochlorid-lsopropanolat, F. 102-105° (Zers.);
von 3-(3-Chlorpropyl)-5-fluorindol mit 4-(5-Fluorindol-3-yl)-piperidin :
   3-[1-(3-(5-Fluor-indol-3-yl)-propyl)-4-piperidyl]-5-fluor-indol, Hydrochlorid-Hemihydrat, F. 164-165°;
von 3-(3-Chlorpropyl)-5-fluorindol mit 4-(6-Fluorindol-3-yl)-piperidin :
   3-[1-(3-(5-Fluor-indol-3-yl)-propyl)-4-piperidyl]-6-fluor-indol, Hydrochlorid-Hydrat, F. 274-278°;
von 3-(3-Chlorpropyl)-5-fluorindol mit 4-(4-Fluorindol-3-yl)-piperidin :
   3-[1-(3-(5-Fluor-indol-3-yl)-propyl)-4-piperidyl]-4-fluor-indol, Hydrochlorid, F. 269-270°;
von 3-(3-Chlorpropyl)-indol-5-carbonsäuremethylester mit 4-(5-Methoxycarbonylindol-3-yl)-piperidin:
   3-[1-(3-(5-Methoxycarbonyl-indol-3-yl)-propyl)-4-piperidyl]-indol-5-carbonsäuremethylester;
von 3-(3-Chlorpropyl)-indol-5-carboxamid mit 4-(5-Cyanindol-3-yl)-piperidin:
   3-[1-(3-(5-Carbamoyl-indol-3-yl)-propyl)-4-piperidyl]-5-cyanindol, Hydrat, F. 102-104° (Zers.).

### Beispiel 4

Analog Beispiel 2 erhält man durch Verseifung von 3-[1-(3-(5-Methoxycarbonyl-indol-3-yl)-propyl)-4-piperidyl]-indol-5-carbonsäuremethylester die 3-[1-(3-(5-Carboxy-indol-3-yl)-propyl)-4-piperidyl]-indol-5-carbonsäure.

Analog erhält man durch Verseifung der entsprechenden Ester ausgehend
von 3-[1-(3-(5-Methoxycarbonylindol-3-yl)-propyl)-4-piperidyl]-5-methoxyindol:
   3-[1-(3-(5-Carboxyindol-3-yl)-propyl)-4-piperidyl]-5-methoxy-indol;
von 3-[1-(3-(5-Methoxyindol-3-yl)-propyl)-4-piperidyl]-indol-5-carbonsäuremethylester:
   3-[1-(3-(5-Methoxyindol-3-yl)-propyl)-4-piperidyl]-indol-5-carbonsäure;
von 3-[1-(3-(6-Methoxyindol-3-yl)-propyl)-4-piperidyl]-indol-5-carbonsäuremethylester:
   3-[1-(3-(6-Methoxyindol-3-yl)-propyl)-4-piperidyl]-indol-5-carbonsäure;
von 3-[1-(3-(4-Methoxyindol-3-yl)-propyl)-4-piperidyl]-indol-5-carbonsäuremethylester:
   3-[1-(3-(4-Methoxyindol-3-yl)-propyl)-4-piperidyl]-indol-5-carbonsäure;
von 3-[1-(3-(5-Methoxycarbonyl-indol-3-yl)-propyl)-4-piperidyl]-indol-5-carboxamid:
   3-[1-(3-(5-Carboxy-indol-3-yl)-propyl)-4-piperidyl]-indol-5-carboxamid, Hydrochlorid, F. 278-280°.

### Beispiel 5

2,1 g 3-[1-(4-(5-Methoxy-indol-3-yl)-butyl)-4-piperidyl]-indol-5-carbonsäure werden in 100 ml N-Methylpyrrolidin suspendiert. Anschließend fügt man 3,2 g 2-Chlor-1-methyl-pyridiniummethan-sulfonat hinzu und rührt bei Raumtemperatur 12 Stunden. In die entstandene Lösung leitet man bis zur Sättigung getrocknetes NH₃-Gas ein und rührt erneut 10 Stunden. Nach üblicher Aufarbeitung erhält man 3-[1-(4-(5-Methoxy-indol-3-yl)-butyl)-4-piperidyl]-indol-5-carboxamid.

Analog erhält man durch Amidierung der nachfolgenden Carbonsäuren mit 2-Chlor-1-methyl-pyridiniummethansulfonat:
von 3-[1-(4-(6-Methoxyindol-3-yl)-butyl)-4-piperidyl]-indol-5-carbonsäure:
   3-[1-(4-(6-Methoxyindol-3-yl)-butyl)-4-piperidyl]-indol-5-carboxamid;
von 3-[1-(4-(4-Methoxyindol-3-yl)-butyl)-4-piperidyl]-indol-5-carbonsäure:
   3-[1-(4-(4-Methoxyindol-3-yl)-butyl)-4-piperidyl]-indol-5-carboxamid;
von 3-[1-(3-(5-Carboxy-indol-3-yl)-propyl)-4-piperidyl]-indol-5-carbonsäure das 3-[1-(3-(5-Carbamoyl-indol-3-yl)-propyl)-4-piperidyl]-indol-5-carboxamid.
von 3-[1-(3-(5-Carboxy-indol-3-yl)-propyl)-4-piperidyl]-5-methoxyindol :
   3-[1-(3-(5-Carbamoylindol-3-yl)-propyl)-4-piperidyl]-5-methoxy-indol;
von 3-[1-(3-(5-Methoxyindol-3-yl)-propyl)-4-piperidyl]-indol-5-carbonsäure:
   3-[1-(3-(5-Methoxyindol-3-yl)-propyl)-4-piperidyl]-indol-5-carboxamid;
von 3-[1-(3-(6-Methoxyindol-3-yl)-propyl)-4-piperidyl]-indol-5-carbonsäure:
   3-[1-(3-(6-Methoxyindol-3-yl)-propyl)-4-piperidyl]-indol-5-carboxamid;
von 3-[1-(3-(4-Methoxyindol-3-yl)-propyl)-4-piperidyl]-indol-5-carbonsäure:
   3-[1-(3-(4-Methoxyindol-3-yl)-propyl)-4-piperidyl]-indol-5-carboxamid.

### Beispiel 6

Eine Lösung von 3,9 g 3-[1-(4-(5-Carboxy-indol-3-yl)-butyl)-4-piperidyl]-indol-5-carbonsäure in 250 ml DMF wird mit 1 g N-Methylmorpholin versetzt. Unter Rühren gibt man eine Lösung von zwei Äquivalenten tert.-Butylamin in 5 ml DMF, 1,3 g 1-Hydroxybenzo-triazol sowie eine Lösung von 1,9 g N-(3-Dimethylaminopropyl)-N'-ethyl-carbodiimid-hydrochlorid in 20 ml DMF hinzu. Man rührt 16 Stunden bei Raumtemperatur und dampft das Filtrat ein. Nach üblicher Aufarbeitung erhält man 3-[1-(4-(5-N-tert.-Butylcarbamoyl-indol-3-yl)-butyl)-4-piperidyl]-indol-5-N-tert.-butyl-carboxamid.

Analog erhält man durch Umsetzung mit tert.-Butylamin ausgehend
von 3-[1-(4-(5-Carboxyindol-3-yl)-butyl)-4-pi peddyl]-5-methoxy-indol:
   3-[1-(4-(5-N-tert.-Butylcarbamoylindol-3-yl)-butyl)-4-piperidyl]-5-methoxy-indol;
von 3-[1-(4-(5-Carboxy-indol-3-yl)-butyl)-4-piperidyl]-indol-5-carboxamid:
   3-[1-(4-(5-N-tert.-Butylcarbamoyl-indol-3-yl)-butyl)-4-piperidyl]-indol-5-carboxamid;
von 3-[1-(3-(5-Carboxy-indol-3-yl)-propyl)-4-piperidyl]-5-methoxy-indol:
   3-[1-(3-(5-N-tert.-Butylcarbamoylindol-3-yl)-propyl)-4-piperidyl]-5-methoxy-indol.

### Beispiel 7

Eine Gemisch von 1,6 g 3-[1-(4-(Indol-3-yl)-butyl)-4-piperidyl]-5-methoxyindol [herstellbar nach Beispiel 1], 1,8 g Pyridinhydrochlorid sowie 50 ml Pyridin wird 3 Stunden gekocht. Man kühlt ab, dampft ein, arbeitet wie üblich auf und erhält 3-[1-(4-(Indol-3-yl)-butyl)-4-piperidyl]-5-hydroxyindol, F. 178-180°.

Analog erhält man
aus 3-[1-(4-(5-Methoxy-indol-3-yl)-butyl)-4-piperidyl]-indol:
   3-[1-(4-(5-Hydroxy-indol-3-yl)-butyl)-4-piperidyl]-indol;
aus 3-[1-(4-(5-Methoxy-indol-3-yl)-butyl)-4-piperidyl]-5-methoxyindol:
   3-[1-(4-(5-Hydroxy-indol-3-yl)-butyl)-4-piperidyl]-5-hydroxyindol;
aus 3-[1-(4-(6-Methoxy-indol-3-yl)-butyl)-4-piperidyl]-5-hydroxyindol:
   3-[1-(4-(6-Hydroxy-indol-3-yl)-butyl)-4-piperidyl]-5-hydroxyindol;
aus 3-[1-(3-(Indol-3-yl)-propyl)-4-piperidyl]-5-methoxyindol:
   3-[1-(3-(Indol-3-yl)-propyl)-4-piperidyl]-5-hydroxyindol;
aus 3-[1-(3-(5-Methoxy-indol-3-yl)-propyl)-4-piperidyl]-5-methoxyindol:
   3-[1-(3-(5-Hydroxy-indol-3-yl)-propyl)-4-piperidyl]-5-hydroxyindol.

### Beispiel 8

Zu einer Suspension von 0,6 g Lithiumaluminiumhydrid in 20 ml THF wird unter Rühren bei Raumtemperatur eine Lösung von 3,6 g 3-[1-(4-(5-Methoxycarbonyl-indol-3-yl)-butyl)-4-piperidyl]-indol in 40 ml THF tropfenweise zugegeben. Anschließend rührt man eine weitere Stunde bei 25°, fügt 20 ml verdünnte Natronlauge hinzu, filtriert und arbeitet das Filtrat wie üblich auf. Man erhält 3-[1-(4-(5-Hydroxymethyl-indol-3-yl)-butyl)-4-piperidyl]-indol.

Analog erhält man durch Reduktion
von 3-[1-(4-(5-Methoxycarbonylindol-3-yl)-butyl)-4-piperidyl]-5-methoxyindol:
   3-[1-(4-(5-Hydroxymethylindol-3-yl)-butyl)-4-piperidyl]-5-methoxyindol;
von 3-[1-(4-(5-Methoxycarbonyl-indol-3-yl)-butyl)-4-piperidyl]-indol-5-carbonsäuremethylester:
   3-[1-(4-(5-Hydroxymethyl-indol-3-yl)-butyl)-4-piperidyl]-5-hydroxymethylindol;
von 3-[1-(3-(5-Methoxycarbonylindol-3-yl)-propyl)-4-piperidyl]-5-methoxyindol:
   3-[1-(3-(5-Hydroxymethylindol-3-yl)-propyl)-4-piperidyl]-5-methoxyindol
von 3-[1-(3-(5-Methoxycarbonyl-indol-3-yl)-propyl)-4-piperidyl]-indol-5-carboxamid:
   3-[1-(3-(5-Hydroxymethylindol-3-yl)-propyl)-4-piperidyl]-indol-5-carboxamid.

### Beispiel 9

In eine siedende Lösung von 2,5 g 3-[1-(4-(5-Carboxyindol-3-yl)-butyl)-4-piperidyl]-5-fluorindol in 50 ml absolutem Methanol wird 2 Stunden HCI-Gas eingeleitet. Anschließend kocht man eine weitere Stunde, arbeitet wie üblich auf und erhält 3-[1-(4-(5-Methoxy-carbonylindol-3-yl)-butyl)-4-piperidyl]-5-fluor indol.

### Beispiel 10

Analog Beispiel 1 erhält man durch Umsetzung von 3-(4-Chlorbutyl)-indol [erhältlich durch Umsetzung von Indol mit 4-Chlorbutyryl-chlorid zu 3-(4-Chlorbutyryl)-indol und anschließende Reduktion mit Diboran] mit 4-(Indol-3-yl)-1,2,5,6-tetrahydropyridin [erhältlich durch Umsetzung von N-BOC-4-Piperidon mit Indol und anschließende Dehydratisierung sowie Abspaltung der Schutzgruppe] in 200 ml Acetonitril nach üblicher Aufarbeitung das 3-[1-(4-(lndol-3-yl)-butyl)-(1,2,5,6-tetrahydropyrid-4-yl)]-indol, Hydrochlorid, F. 190-192°.

Analog erhält man durch Umsetzung
von 3-(4-Chlorbutyl)-indol-5-carbonsäuremethylester mit 4-(5-Methoxyindol-3-yl)-1 ,2,5,6-tetrahydropyridin:
   3-[1-(4-(5-Methoxycarbonylindol-3-yl)-butyl)-(1,2,5,6-tetra-hydropyrid-4-yl)]-5-methoxyindol;
von 3-(4-Chlorbutyl)-indol mit 4-(5-Methoxyindol-3-yl)-1,2,5,6-tetrahydropyridin:
   3-[1-(4-(Indol-3-yl)-butyl)-(1,2,5,6-tetrahydropyrid-4-yl)]-5-methoxyindol;
von 3-(4-Chlorbutyl)-5-methoxyindol mit 4-(5,6-Methylendioxyindol-3-yl)-1,2,5,6-tetrahydropyridin:
   3-[1-(4-(5-Methoxyindol-3-yl)-butyl)-(1,2,5,6-tetrahydropyrid-4-yl)]-5,6-methylendioxyindol;
von 3-(4-Chlorbutyl)-5-methoxyindol mit 4-(5-Methoxyindol-3-yl)-1,2,5,6-tetrahydropyridin:
   3-[1-(4-(5-Methoxy-indol-3-yl)-butyl)-(1,2,5,6-tetrahydropyrid-4-yl)]-5-methoxyindol;
von 3-(4-Chlorbutyl)-indol mit 4-(3-lndolyl)-1,2,5,6-tetrahydropyridin:
   3-[1-(4-(Indol-3-yl)-butyl)-(1,2,5,6-tetrahydropyrid-4-yl)]-indol;
von 3-(4-Chlorbutyl)-5-methoxyindol mit 4-(5-Hydroxyindol-3-yl)-1,2,5,6-tetrahydropyridin:
   3-[1-(4-(5-Methoxy-indol-3-yl)-butyl)-(1,2,5,6-tetrahydropyrid-4-yl)]-5-hydroxyindol;
von 3-(4-Chlorbutyl)-5-cyanindol mit 4-(5-Carbamoylindol-3-yl)-1,2,5,6-tetrahydropyridin:
   3-[1-(4-(5-Cyan-indol-3-yl)-butyl)-(1,2,5,6-tetrahydropyrid-4-yl)]-indol-5-carboxamid;
von 3-(4-Chlorbutyl)-5-cyanindol mit 4-(5-Cyanindol-3-yl)-1,2,5,6-tetrahydropyridin:
   3-[1-(4-(5-Cyan-indol-3-yl)-butyl)-(12,5,6-tetrahydropyrid-4-yl)]-5-cyanindol;
von 3-(4-Chlorbutyl)-5-methoxycarbonylindol mit 4-(5-Carbamoyl-indol-3-yl)-1 ,2,5,6-tetrahydropyridin:
   3-[1-(4-(5-Methoxycarbonyl-indol-3-yl)-butyl)-(1,2,5,6-tetrahydropyrid-4-yl)]-indol-5-carboxamid;
von 3-(4-Chlorbutyl)-indol-5-carboxamid mit 4-(5-Carbamoylindol-3-yl)-1,2,5,6-tetrahydropyridin:
   3-[1-(4-(5-Carbamoyl-indol-3-yl)-butyl)-(1,2,5,6-tetrahydropyrid-4-yl)]-indol-5-carboxamid;
von 3-(4-Chlorbutyl)-5-fluorindol mit 4-(5-Fluorindol-3-yl)-1,2,5,6-tetra-hydropyridin:
   3-[1-(4-(5-Fluor-indol-3-yl)-butyl)-(1,2,5,6-tetrahydropyrid-4-yl)]-5-fluor-indol;
von 3-(4-Chlorbutyl)-indol-5-carbonsäuremethylester mit 4-(5-Methoxycarbonylindol-3-yl)-1,2,5,6-tetrahydropyridin:
   3-[1-(4-(5-Methoxycarbonyl-indol-3-yl)-butyl)-(1,2,5,6-tetrahydropyrid-4-yl)]-indol-5-carbonsäuremethylester;
von 3-(4-Chlorbutyl)-indol-5-carboxamid mit 4-(5-Cyanindol-3-yl)-1,2,5,6-tetrahydropyridin:
   3-[1-(4-(5-Carbamoyl-indol-3-yl)-butyl)-(1,2,5,6-tetrahydropyrid-4-yl)]-5-cyanindol.

### Beispiel 11

Analog Beispiel 1 erhält man ausgehend von 3-(2-Chlorethyl)-2-methyl-5-methoxy-indol [erhältlich durch Umsetzung von 2-Methyl-5-methoxyindol mit 2-Chloracetylchlorid zu 3-(2-Chloracetyl)-2-methyl-5-methoxyindol und anschließende Reduktion mit Diboran] sowie 1,0 g 4-(5-Fluorindol-3-yl)-piperidin [erhältlich durch Umsetzung von N-BOC-4-Piperidon mit 5-Fluorindol, anschließende Dehydratisierung und Hydrierung der entstehenden Doppelbindung sowie Abspaltung der Schutzgruppe] nach üblicher Aufarbeitung das 3-[1-(2-(2-Methyl-5-methoxy-indol-3-yl)-ethyl)-4-piperidyl]-5-fluor-indol, Hydrochlorid, Rf = 0,31.

Analog erhält man durch Umsetzung
von 3-(2-Chlorethyl)-indol mit 4-(5-Fluor-indol-3-yl)-piperidin :
   3-[1-(2-lndol-3-yl)-ethyl)-4-piperidyl]-5-fluorindol, Hydrochlorid, Rf = 0,20;
von 3-(2-Chlorethyl)-indol mit 4-(4-Fluor-indol-3-yl)-piperidin:
   3-[1-(2-(lndol-3-yl)-ethyl)-4-pipetdyl]-4-fluorindol, Hydrochlorid, F. 297°;
von 3-(2-Chlorethyl)-2-methyl-5-methoxyindol mit 4-(4-Fluor-indol-3-yl)-piperidin:
   3-[1-(2-(2-Methyl-5-methoxy-indol-3-yl)-ethyl)-4-piperidyl]-4-fluorindol, Hydrochlorid, F. 215°;
von 3-(2-Chlorethyl)-indol mit 4-(5-Methoxy-indol-3-yl)-piperidin:
   3-[1-(2-(Indol-3-yl)-ethyl)-4-piperidyl]-5-methoxy-indol;
von 3-(2-Chlorethyl)-2-methyl-5-methoxyindol mit 4-(4-Methoxy-indol-3-yl)-piperidin:
   3-[1-(2-(2-Methyl-5-methoxy-indol-3-yl)-ethyl)-4-piperidyl]-4-methoxy-indol.

### Beispiel 12

0,4 g 3-[1-((5-Fluor-indol-3-yl)-methylcarbonyl)-4-piperidyl]-5-fluorindol [erhältlich durch Umsetzung von 5-Fluorindol mit 2-Chloracetylchlorid zu 3-(2-Chloracetyl)-5-methoxyindol und anschließende Reaktion mit 4-(5-Fluor-indol-3-yl)-piperidin] werden in 30 ml THF gelöst und tropfenweise bei Raumtemperatur mit 1,3 Äquivalenten NaAl(OCH₂CH₂OCH₃)₂H₂, gelöst in 10 ml Toluol, versetzt. Man rührt 2 Std. bei Raumtemperatur, arbeitet anschließend wie üblich auf und erhält 3-[1-(2-(5-Fluor-indol-3-yl)-ethyl)-4-piperidyl]-5-fluorindol, Hydrochlorid, Rf = 0,27.

Analog erhält man durch Reduktion mit NaAl(OCH₂CH₂OCH₃)₂H₂:
aus 3-[1-(2-(5-Fluor-indol-3-yl)-ethylcarbonyl)-4-piperidyl]-4-fluorindol:
   3-[1-(3-(5-Fluor-indol-3-yl)-propyl)-4-piperidyl]-4-fluor-indol;
aus 3-[1-((5-Fluor-indol-3-yl)-methylcarbonyl)-4-piperidyl]-4-fluorindol:
   3-[1-(2-(5-Fluor-indol-3-yl)-ethyl)-4-piperidyl]-4-fluorindol, Hydrochlorid, F. 260°.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat werden in 3 1 zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. 3-Indolylpiperidine der Formel I 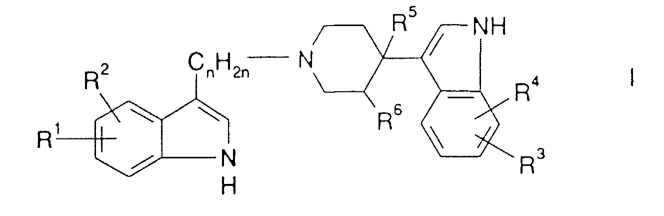 worin
R¹, R², R³ und R⁴ jeweils unabhängig voneinander H, A, OH, OA, F, Cl, Br, I, CN, CF₃, COOH, CONH₂, CONHA, CONA₂ oder COOA, oder
R¹ und R² sowie R³ und R⁴ jeweils zusammen auch Methylendioxy,
R⁵ H oder OH,
R⁶ H oder
R⁵ und R⁶ auch gemeinsam eine Bindung,
A Alkyl mit 1 bis 6 C-Atomen
n 2, 3, 4, 5 oder 6
bedeuten,
sowie deren physiologisch unbedenkliche Salze.

2. (a) 3-(1-(3-(5-Carbamoyl-3-indolyl)-propyl)-piperid-4-yl)-indol-5-carboxamid;
(b) 3-(1-(3-(5-Carbamoyl-3-indolyl)-propyl)-piperid-4-yl)-indol-5-carbonitril;
(c) 3-(1-(4-(5-Methoxycarbonyl-3-indolyl)-butyl)-piperid-4-yl)-5-methoxy-indol;
(d) 3-(1-(3-(5-Ethoxycarbonyl-3-indolyl)-propyl)-piperid-4-yl)-indol-5-carboxamid;
(e) 3-(1-(4-(5-Cyan-3-indolyl)-butyl)-piperid-4-yl)-indol-5-carbonitril
gemäß Anspruch 1 sowie deren physiologisch unbedenkliche Salze.

3. Verfahren zur Herstellung von 3-lndolylpiperidinderivaten der Formel I sowie von deren Salzen, dadurch gekennzeichnet, daß man eine Verbindung der Formel II 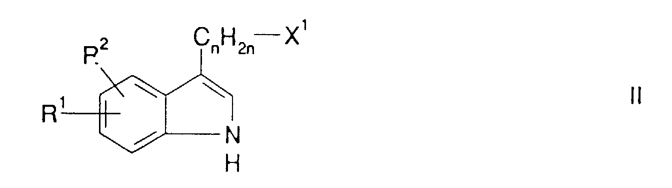 worin
X¹ X oder NH₂ und
X Cl, Br, I, OH oder eine reaktionsfähig funktionell abgewandelte OH-Gruppe bedeuten und
R¹, R² und n die angegebenen Bedeutungen haben, mit einer Verbindung der Formel III 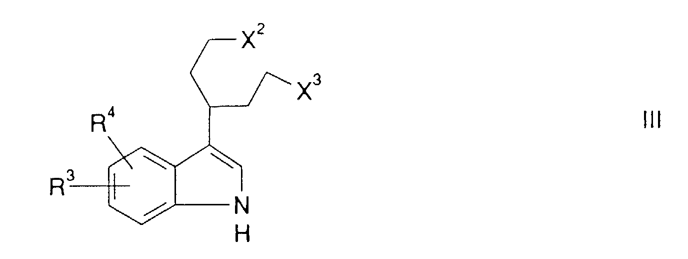 worin
X² und X³ gleich oder verschieden sein können und, falls X¹ = NH₂ ist, jeweils X, andernfalls zusammen NH bedeuten und
R³ und R⁴ die angegebenen Bedeutungen haben,
umsetzt,
oder daß man eine Verbindung der Formel IV 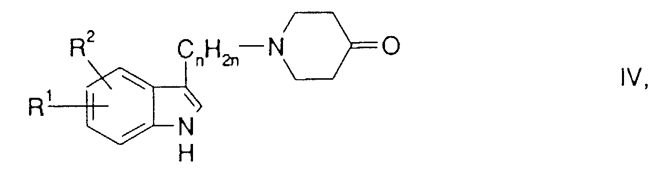 worin R¹, R² und n die angegebenen Bedeutungen haben,
mit einem Indol der Formel V 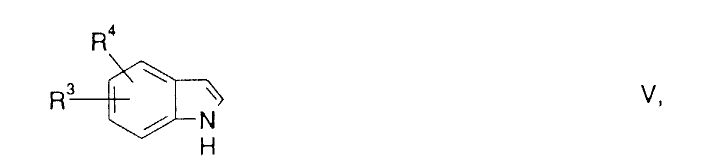 worin R³ und R⁴ die angegebenen Bedeutungen besitzen,
umsetzt,
oder daß man eine Verbindung der Formel I, in der R⁵ OH und R⁶ H bedeutet, durch Dehydratisierung in eine andere Verbindung der Formel I umwandelt,
oder daß man eine sonst der Formel I entsprechende Verbindung, die jedoch anstelle eines oder mehrerer Wasserstoffatome eine oder mehrere reduzierbare Gruppe(n) und/oder eine oder mehrere zusätzliche C-C-und/oder C-N-Bindung(en) enthält, mit einem reduzierenden Mittel behandelt
oder daß man eine sonst der Formel I entsprechende Verbindung, die jedoch anstelle eines oder mehrerer Wasserstoffatome eine oder mehrere solvolysierbare Gruppe(n) enthält, mit einem solvolysierenden Mittel behandelt
und/oder daß man einen Rest R¹, R², R³ und/oder R⁴ durch Veresterung, Verseifung, Veretherung, Etherspaltung, vollständige oder partielle Hydrolyse oder durch Alkylierung in (einen) andere(n) Rest(e) R¹, R², R³ und/oder R⁴ umwandelt
und/oder daß man eine erhaltende Base oder Säure der Formel I durch Behandeln mit einer Säure oder Base in eines ihrer Salze umwandelt.

4. Verfahren zur Herstellung pharmazeutischer Zubereitungen, dadurch gekennzeichnet, daß man eine Verbindung der Formel I gemäß Anspruch 1 und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der allgemeinen Formel I gemäß Anspruch 1 und/oder einem ihrer physiologisch unbedenklichen Salze.

6. Verwendung von Verbindungen der Formel I nach Anspruch 1 oder von deren physiologisch unbedenklichen Salzen zur Herstellung eines Arzneimittels.

## Claims

1. 3-Indolylpiperidines of the formula I 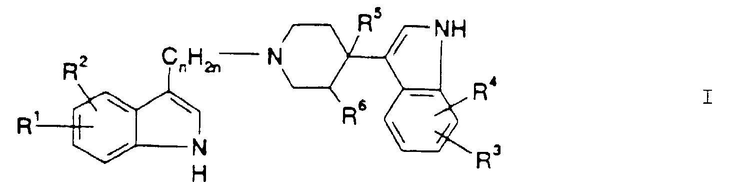 wherein
R¹, R², R³ and R⁴ are in each case independently of one another H, A, OH, OA, F, Cl, Br, I, CN, CF₃, COOH, CONH₂, CONHA, CONA₂ or COOA, or
R¹ and R² and also R³ and R⁴ in each case together are also methylenedioxy,
R⁵ is H or OH,
R⁶ is H or
R⁵ and R⁶ together are also a bond,
A is alkyl having 1 to 6 C atoms and
n is 2, 3, 4, 5 or 6,
and to their physiologically acceptable salts.

2. (a) 3-(1-(3-(5-carbamoyl-3-indolyl)propylpiperid-4-yl)indole-5-carboxamide;
(b) 3-(1-(3-(5-carbamoyl-3-indolyl)propyl)piperid-4-yl)indole-5-carbonitrile;
(c) 3-(1-(4-(5-methoxycarbonyl-3-indolyl)butyl)piperid-4-yl)-5-methoxyindole;
(d) 3-(1-(3-(5-ethoxycarbonyl-3-indolyl)propyl)piperid-4-yl)indole-5-carboxamide;
(e) 3-(1-(4-(5-cyano-3-indolyl)butylpiperid-4-yl)indole-5-carbonitrile
according to Claim 1 and their physiologically acceptable salts.

3. Process for the preparation of 3-indolylpiperidine derivatives of the formula I, and their salts, characterized in that a compound of the formula II 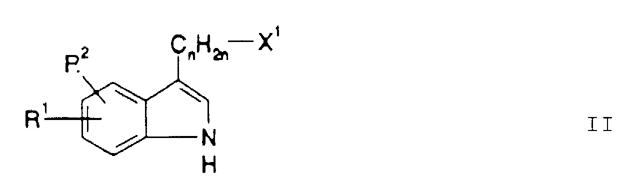 wherein
X¹ is X or NH₂,
X is Cl, Br, I, OH or an OH group functionally modified to form a reactive group, and
R¹, R² and n are as defined, is reacted with a compound of the formula III 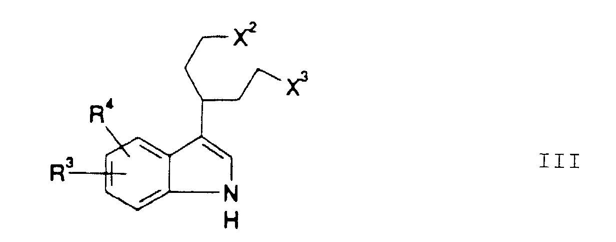 wherein
X² and X³
can be identical or different and are each X if X¹ = NH₂ or are together NH in other cases, and
R³ and R⁴ are as defined,
or in that a compound of the formula IV 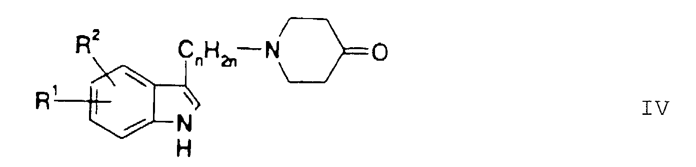 wherein R¹, R² and n are as defined, is reacted with an indole of the formula V 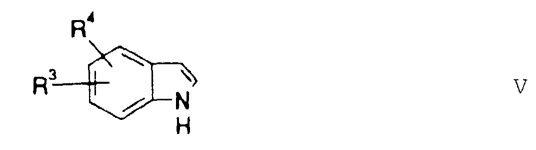 wherein R³ and R⁴ are as defined,
or in that a compound of the formula I in which R⁵ is OH and R⁶ is H is converted into another compound of the formula I by dehydration,
or in that a compound which has the formula I except that one or more hydrogen atoms have been replaced by one or more reducible groups and/or one or more additional C-C and/or C-N bonds is treated with a reducing agent,
or in that a compound which has the formula I except that one or more hydrogen atoms have been replaced by one or more solvolyzable groups is treated with a solvolyzing agent,
and/or in that a radical R¹, R², R³ and/or R⁴ is converted into an(other) radical(s) R¹, R², R³ and/or R⁴ by esterification, hydrolysis, etherification, ether cleavage, complete or partial hydrolysis or by alkylation
and/or in that a resulting base or acid of the formula I is converted into one of its salts by treatment with an acid or base.

4. Process for the manufacture of pharmaceutical preparations, characterized in that a compound of the formula I according to Claim 1 and/or one of its physiologically acceptable salts are converted into a suitable dosage form together with at least one solid, liquid or semiliquid excipient or adjunct.

5. Pharmaceutical preparation, characterized in that it contains at least one compound of the general formula I according to Claim 1 and/or one of its physiologically acceptable salts.

6. Use of compounds of the formula I according to Claim 1, or their physiologically acceptable salts, for the manufacture of a drug.

## Revendications

1. Les 3-indolylpipéridines de formule I 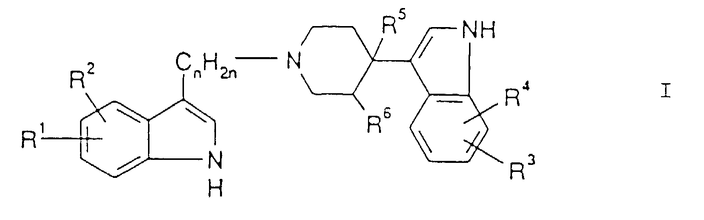 où
R¹, R², R³ et R⁴ représentent, indépendamment les uns des autres, H, A, OH, OA, F, Cl, Br, I, CN, CF₃, COOH, CONH₂, CONHA, CONA₂ ou COOA, ou
R¹ et R², ainsi que R³ et R⁴ représentent ensemble également le groupe méthylènedioxy,
R⁵ représente H ou OH,
R⁶ H ou
R⁵ et R⁶ représentent ensemble également une liaison,
A représente un alkyle comportant 1 à 6 atomes de C,
n est égal à 2, 3, 4, 5 ou 6,
ainsi que leurs sels physiologiquement acceptables.

2. (a) le 3-(1-(3-(5-carbamoyl-3-indolyl)propyl)pipérid-4-yl)indol-5-carboxamide ;
(b) le 3-(1-(3-(5-carbamoyl-3-indolyl)propyl)pipérid-4-yl)indol-5-carbonitrile ;
(c) le 3-(1-(4-(5-méthoxycarbonyl-3-indolyl)butyl)pipérid-4-yl)-5-méthoxyindol ;
(d) le 3-(1-(3-(5-éthoxycarbonyl-3-indolyl)propyl)pipérid-4-yl)indol-5-carboxamide ;
(e) le 3-(1-(4-(5-cyano-3-indolyl)butyl)pipérid-4-yl)indol-5-carbonitrile ;
selon la revendication 1, ainsi que leurs sels physiologiquement acceptables.

3. Procédé pour la préparation des dérivés des 3-indolpipéridines de formule I ainsi que de leurs sels, caractérisé en ce que l'on fait réagir un composé de formule II 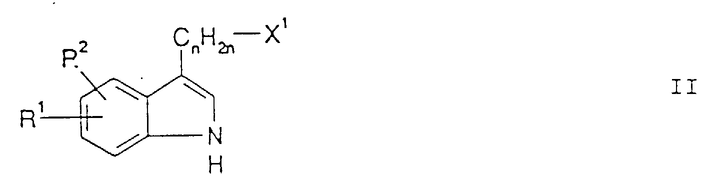 où
X¹ représente X ou NH₂ et
X Cl, Br, I, OH ou un groupe OH réactif modifié fonctionnellement et
R¹, R² et n ont les significations indiquées, avec un composé de formule III 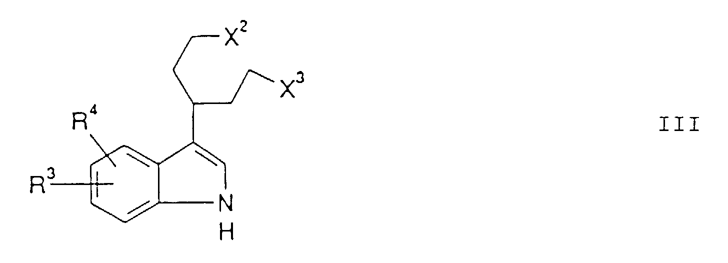
où
X² et X³ peuvent être identiques ou différents et, dans le cas où X1 = NH₂, ils représentent X, sinon ils représentent ensemble NH et
R³ et R⁴ ont les significations indiquées,
ou en ce que l'on fait réagir un composé de formule IV 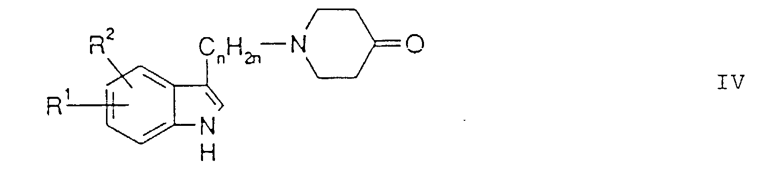 où R¹, R² et n ont les significations indiquées, avec un indol de formule V 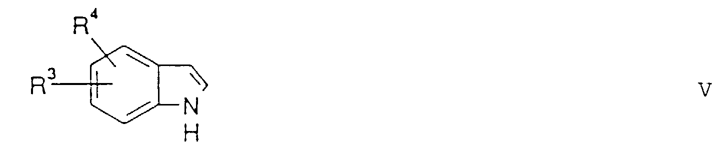 où R³ et R⁴ ont les significations indiquées,
ou en ce que l'on transforme par déshydratation un composé de formule I dans laquelle R⁵ représente OH et R⁶ représente H en un autre composé de formule I,
ou en ce que l'on traite un composé correspondant à la formule I, mais contenant à la place d'un ou plusieurs atome(s) d'hydrogène un ou plusieurs groupe(s) réductible(s) et/ou une ou plusieurs liaison(s) C-C et/ou C-N supplémentaire(s), avec un agent réducteur,
ou en ce que l'on traite un composé correspondant à la formule I, mais contenant à la place d'un ou plusieurs atome(s) d'hydrogène un ou plusieurs groupe (s) solvolysable(s), avec un agent solvolysant,
et/ou en ce que l'on transforme un reste R¹, R², R³ et/ou R⁴ par estérification, saponification, éthérification, clivage de l'éther, hydrolyse totale ou partielle ou par alcoylation en un autre ou en d'autres reste(s) R¹, R², R³ et/ou R⁴,
et/ou en ce que l'on transforme la base ou l'acide de formule I obtenue par traitement avec un acide ou une base en l'un de leurs sels.

4. Procédé pour la fabrication de préparations pharmaceutiques, caractérisé en ce que l'on met un composé de formule I selon la revendication 1 et/ou l'un de ses sels physiologiquement acceptables, associé à au moins un support ou un adjuvant solide, liquide ou semi-liquide sous une forme de dosage appropriée.

5. Préparation pharmaceutique caractérisée en ce qu'elle contient au moins un composé de formule générale I selon la revendication 1 et/ou l'un de ses sels physiologiquement acceptables.

6. Utilisation des composés de formule I selon la revendication 1 ou de leurs sels physiologiquement acceptables pour la fabrication d'un médicament.
